**Europäisches Patentamt**

**European Patent Office**

**Office européen des brevets**

(19)

(11) **EP 1 227 074 A1**

(12) **EUROPEAN PATENT APPLICATION**

(43) Date of publication:
**31.07.2002 Bulletin 2002/31**

(51) Int Cl.⁷: **C07C 1/04**, C07C 2/04,
C07C 2/76, C10L 3/06,
C10J 3/00, H05H 1/00

(21) Application number: **02250601.8**

(22) Date of filing: **29.01.2002**

| | |
|---|---|
| (84) Designated Contracting States:<br>**AT BE CH CY DE DK ES FI FR GB GR IE IT LI LU<br>MC NL PT SE TR**<br>Designated Extension States:<br>**AL LT LV MK RO SI**<br><br>(30) Priority: **29.01.2001 JP 2001059205**<br><br>(71) Applicant: **Hatanaka, Takefumi<br>Tokyo 193-0836 (JP)** | (72) Inventor: **Hatanaka, Takefumi<br>Tokyo 193-0836 (JP)**<br><br>(74) Representative:<br>**Lyndon-Stanford, Edward Willoughby Brooke<br>MARKS & CLERK,<br>57/60 Lincoln's Inn Fields<br>London WC2A 3LS (GB)** |

(54) **Method and system for producing heavier hydrocarbons from solid carbon and water**

(57)    A method and system for producing heavier hydrocarbons from solid carbon materials and water are disclosed as including an arc plasma reactor (APR) which has arc discharge electrodes ((36, 38, 40) and a large number of minute arc passages (35) formed in the solid carbon materials filled in the plasma reactor. Feed water is converted into steam in the plasma reactor and the steam is fed through the minute arc passages in which steam reacts with the solid carbon materials in the presence of arc plasmas to produce synthesis gas. The synthesis gas is converted into methane and a portion of methane is converted into acetylene. A mixture of methane and acetylene is reacted in the presence of a solid superacid catalyst into isobutene, which in turn is converted into heavier hydrocarbons in an oligomerization reactor. The hydrocarbons are distilled into gasoline-range and jet fuel-range liquid fuels.

FIG. 2

EP 1 227 074 A1

**Description**

BACKGROUND OF THE INVENTION

1. Field of the Invention

This invention relates to methods and systems for producing heavier hydrocarbons from natural gas and, more particularly, to a method and apparatus for producing heavier hydrocarbons from synthesis gas converted from natural gas.

2. Description of the Related Art

Extensive research and development works have been undertaken to produce clean hydrocarbons from natural gas in a gas to liquid oil conversion method, i.e. a so-called GTL system.

U.S. Patent No. 4,579,985, U.S. Patent No. 4,579, 986, U.S. Patent No. 4,628,133, U.S. Patent No. 4,709,108, U.S. Patent No. 4,714,796, U.S. Patent No. 4,721,828, U.S. Patent No. 4,727,205, U.S. Patent No. 5,037,856, U.S. Patent No. 5,723,505, U.S. Patent No. 5,763,716, U.S. Patent No. 6,011,073 and U.S. Patent No. 6,085,512 disclose technologies for converting natural gas into heavier hydrocarbons. In these prior art technologies, it is a usual practice to carry out steam reforming of natural gas to form synthesis gas. However, natural gas encounters high costs in mining, liquefaction and transportation and, so, material cost of natural gas highly increases. Further, since natural gas contains sulfur, which is removed by a large and complex plant which is expensive to manufacture, with a resultant increase in size of the heavier hydrocarbon production plant and an increase in a production cost. Further, even if a raw gas is desulferized with the use of a desulferization apparatus, it is difficult co completely remove the sulfur content from the raw gas, and the synthesis gas produced from such raw gas contains a minute amount of sulfur compounds which accumulate in the system, resulting in a degradation of the catalysts. For, this reason, catalysts should be replaced with new ones many times in a short operating period, interrupting the operation of the production plant. This is reflected in an inefficient operation of the plant as well as in a remarkable increase in the production cost. Furthermore, the prior art technologies feature the use of a Fischer/Tropsh reaction (F/T reaction method) by which the synthesis gas is converted to the heavier hydrocarbons. During operation of this method, although the reaction product contains a wide range of products such as water, alcohol, hydrocarbons and offgas, the amount of heavier hydrocarbons contained in the reaction product is extremely small and the hydrocarbon production plant has an extremely low production yield.

To address the above issues, U.S. Patent No. 4,433,192, U.S. Patent No. 4,465,893, U.S. Patent No. 4,467,130 and U.S. Patent No. 4,513,164 propose new processes each of which uses a solid superacid catalyst that directly converts natural gas into gasoline-range hydrocarbons. It has also been proposed to further improve such a production process to further increase the production yield of the hydrocarbons as disclosed by U. S. Patent No. 4,973,776. This prior art enables a mixture of methane and acetylene to be converted into isobutene as an intermediate at a high yield rate of 95 %. Then, the intermediate is reacted with oligomerization catalyst to form gasoline-range and jet fuel-range hydrocarbons at a high production yield rate.

Such a two-stage procedure, in which isobutene is formed as an intermediate, is highly effective in increasing a conversion rate of natural gas into hydrocarbons in simplified steps and is superior to the Fischer/Tropsh method. However, the use of natural gas and acetylene gas results in a remarkable increase in the raw material costs. Also, transportation of natural gas and acetylene to a final hydrocarbon production plant requires an extremely high skills with a further increase in transportation cost. Accordingly, it is extremely difficult for producing gasoline-range and jet fuel-range hydrocarbons at a low cost on a mass production basis.

SUMMARY OF THE INVENTION

It is therefore an object of the present invention to provide a method and system for producing gasoline-range and jet fuel-range hydrocarbons from low cost solid carbon materials and water on a mass production basis at a remarkably low cost.

According to one aspect of the present invention, there is provided a method of producing hydrocarbons from solid carbon and feed water, comprising the steps of: preparing an arc plasma reactor having a reactor chamber and arc electrodes located in the reactor chamber; supplying solid carbon particles into the reactor chamber to form a large number of minute arc passages in the solid carbon particles; supplying electric power to the arc electrodes to produce arc discharge plasmas in the minute arc passages, respectively; passing steam through the minute arc passages to cause the carbon particles to react with the steam under the presence of the arc discharge plasmas to produce synthesis gas containing $H_2$ and CO; introducing the synthesis gas into a methanation catalyst of a methanation reactor to synthesize methane; preparing first and second streams of methane; converting the second stream of methane into acetylene; reacting a mixture of the methane and the acetylene in the presence of a solid superacid catalyst to form isobutene; converting the isobutene product in the presence of oligomerization catalyst into hydrocarbons.

According to another aspect of the present invention, there is provided a hydrocarbon production system comprising: an arc plasma reactor having a solid carbon supply port, a feed water supply port, an insulating casing formed with a synthesis gas outlet, an arc plasma chamber formed in the insulating casing, arc electrodes located in the arc plasma chamber, and a plurality of minute arc passages formed in solid carbon particles filled in the arc plasma chamber; a feed water supply pump for supplying feed water into the arc plasma chamber via the feed water supply port to cause the feed water to be converted into steam; an arc power supply for supplying electric power to the arc electrodes to cause arc discharge plasmas to be generated in the minute arc passages, respectively, such that the water is exposed to the arc discharge plasmas to form the steam which reacts with the carbon particles in the presence of the arc discharge plasmas during passing through the minute arc passages to produce synthesis gas containing $H_2$ and CO; and a methanation reactor having a methanation catalyst for converting the synthesis gas into methane; an acetylene synthesizing reactor for converting the methane into acetylene; a solid superacid catalyst reactor for reacting a mixture of the methane and acetylene to synthesize isobutene; and an oligomerization reactor for oligomerizing the isobutene into heavier hydrocarbons.

BRIEF DESCRIPTION OF THE DRAWINGS

The invention, together with objects and advantages thereof, may best be understood by reference to the following description of the presently preferred embodiments together with the accompanying drawings in which:

FIG. 1 is a schematic view of a heavier hydrocarbon production system to carry out a method of the present invention; and
FIG. 2 is an enlarged cross sectional view of an arc plasma reactor shown in FIG. 1.

DETAILED DESCRIPTION OF THE PREFERRED EMBODIMENTS

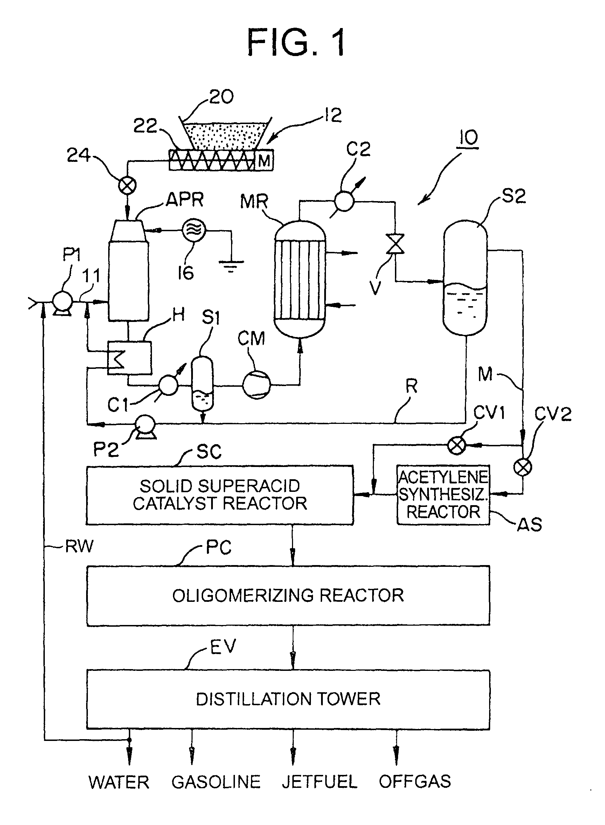

Referring to the drawings, FIG. 1 shows a heavier hydrocarbon production system 10 of a preferred embodiment according to the present invention to carry out a method of the present invention.
In FIG. 1, the heavier hydrocarbon production system 10 is comprised of a solid carbon feed unit 12 which supplies solid carbon materials such as granular, particle, powder or ball-shaped graphite or activated carbon materials, a water feed pump P1 for supplying feed water, an arc plasma reactor APR for converting the solid carbon materials in the presence of steam into a synthesis gas mainly containing hydrogen and carbon monoxide, a heat exchanger H located at a down stream side of the arc plasma reactor APR for cooling the synthesis gas while preheating recycle water, a cooling unit C1 connected to the heat exchanger H for further cooling the synthesis gas to a desired low temperature suitable for subsequent reaction, a liquid/gas separator S1 for separating the synthesis gas and condensed water, a compressor CM for pressuring the synthesis gas, a methanation reactor MR filled with a methanation catalyst to convert the synthesis gas into methane, a cooler C2 for cooling the methane, an expansion valve V for reducing the pressure of the methane, a liquid/gas separating unit S2 for separating the methane and condensed water from each other, a methanation line M, and a recycle line R for recycling condensed water to the arc plasma reactor APR via a pump P2 and the heat exchanger H. The solid carbon materials may also include coal and in this case the heavier hydrocarbon production system 10 must be provided with a known desulferization equipment.
The compressor CM operates for pressurizing the synthesis gas to a value ranging from 15 to 50 atm. The methanation reactor MR is preheated by a circulating heating medium form outside to heat the reactor at a temperature between 250 and 500°C suitable for conversion of the synthesis gas into the methane at the highest efficiency.
As shown in FIG. 1, the hydrocarbon production system 10 further includes first and second flow control valves CV1, CV2 by which first and second streams of methane are produced at respective flow rates. The second stream of methane is delivered to an acetylene synthesizing reactor AS. The acetylene synthesizing reactor AS may includes a microwave reactor which is comprised of a glass tube, through which the second stream of methane flows at a linear velocity of 10 to 15 cm/min, and a microwave generator which irradiates microwaves of 2. 45 GH to the second stream of methane to convert the methane into acetylene with a high selectivity in the order of 95 mol %. The flow control valves CV1, CV2 are controlled to provide the flow rates of the methane in mole ratios of 10-10 : 1. The stream of acetylene synthesized in the reactor AS is then mixed with the first stream of methane delivered through the flow control valve Cv1 to form a mixture of methane and acetylene which is delivered to the solid superacid reactor SC, which is filled with solid superacid catalysts which may be of the types described in U.S. Patent 4,465,893, U.S. Patent No. 5,780,383 and U.S. Patent No. 6,180,556. The solid superacid reactor SC is maintained at a temperature in a range between 20 and 50 °C and at a pressure in a range between 1 and 25 atm by which a mixture of methane and acetylene is converted to isobutene at a selectivity of more than 95 mol %. Then, the isobutene is delivered to an oligomerization reactor PC which is filled with oligomerizing catalysts to achieve oligomerization of the isobutene to produce gasoline-range and jet fuel-range hydrocarbons.
The zeolite catalyst preferred used as the oligomerization catalyst includes the crystalline alumino silicate zeolites having a silica to alumina molar ratio of at least 12. Representative of the ZSM-5 type zeolites are ZSM-5, ZSM-11, ZSM-12, ZSM-23, ZSMA-35 and ZSMA-38. ZSM-5 is disclosed in U. S. Patent No.

3,702,886 and ZSM-11 is disclosed in U.S. Patent No. 3,709,979. Also, see U.S. Patent No. 3,832,449 for ZSM-12; U.S. Patent No. 4,076,842 for ZSM-23; U.S> Patent No. 4,016,145 for ZSM-35 and U.S. Patent No. 4,046,839 for ZSM-38.

The oligomerization reactor PC is preferably maintained at a temperature of 50 to 300 °C and at a pressure of 10 to 50 atm. The hydrocarbon product is then distilled in a distillation tower EV to produce gasoline-range and jet fuel-range hydrocarbons, water and offgas, etc. Water is recycled to the arc plasma reactor APR via a recycle line RW and the pump P1 for reuse.

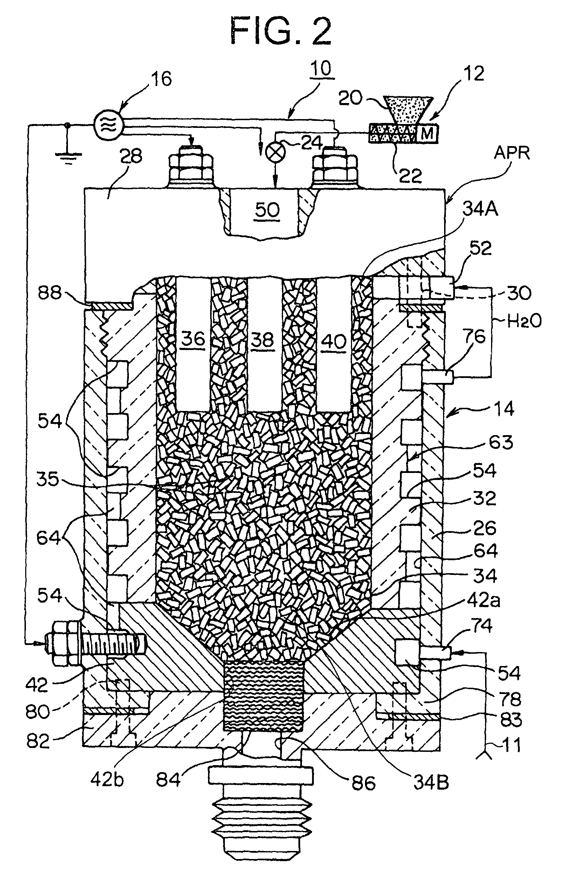

FIG. 2 shows a detailed structure of the arc plasma reactor APR shown in FIG. 1. In FIG. 2, the arc plasma reactor APR includes a thermal reactor unit 14 connected to the solid carbon feed unit 12, and an arc discharge power supply 16. The solid carbon feed unit 12 is comprised of a hopper 20 which stores the solid carbon materials, a screw feeder 22 and a rotary valve 24 to continuously supply the solid carbon particles at a predetermined feed rate. The thermal reactor unit 14 includes a cylindrical outer insulating casing 26 made of heat resistant ceramic, and an inner insulating casing 32 having a cylindrical plasma reaction chamber 34. An insulating electrode holder 28 is coupled to an upper end of the inner insulating casing 32 by means of fixture bolts 30. The plasma reaction chamber 34 has an upstream side formed with a steam generating zone 34A and a downstream side formed with a synthesis gas generating zone 34B. In a practical case, the synthesis gas generating zone 34B occupies a major part of the plasma reaction chamber 34. When the solid carbon materials are supplied into the plasma reaction chamber 34, a large number of minute arc passages 35 are formed between adjacent gaps on the carbon materials through which large number of small arcs are created due to sparks in a uniform manner in the presence of steam which serves as plasma gas. When this occurs, feed water is exposed to a high temperature at the steam generating zone 34A and converted into a stream of steam. The stream of steam flows through the large number of minute arc passages 35 toward the downstream side. During such flow of stream of steam, the steam reacts with the solid carbon materials under the presence of arc plasma to form the synthesis gas containing hydrogen and carbon monoxide according to the reacting formula.

$$C + H_2O \rightarrow CO + H_2 \qquad (1)$$

$$CO + H_2O \rightarrow CO_2 + H_2 \qquad (2)$$

It should be noted here that the hydrogen concentration in the synthesis gas increases as the reaction temperature increases and that the $H_2$/CO ratio can be adjusted to a suitable value for an efficient conversion of the synthesis gas.

The insulating electrode holder 28 supports rod-like arc discharge electrodes 36, 38, 40. An annular disc shaped neutral electrode 42 is located at a lower portion of the insulating casing 32. The neutral electrode 42 has a conical surface 42a and a central opening 42b. The neutral electrode 42 is placed and supported by an electrode holder 78 formed at a bottom of the insulating casing 26 and fixed in place with fixture bolts 80. On the other hand, the electrode holder 28 has a carbon supply port 50 connected to the solid carbon feed unit 12. An upper portion of the outer insulating casing 26 has a feed water supply port 52 formed in the vicinity of upper areas of the arc electrodes 36, 38, 40 for introducing feed water into the steam generating zone 34A. This is advantageous in that feed water serves as coolant for preventing the electrodes 36, 38, 40 from being raised to an excessively high temperature and that feed water is effectively converted into steam which serves as plasma gas for promoting generation of multiple arcs in the synthesis gas generating section 35. Outer peripheries of the inner casing 32 and the neutral electrode 42 are formed with cooling and heat recapturing section 63 composed of annular coolant passages 54, with the adjacent coolant passages being connected to one another through intermediate passages 54. The outer insulating 26 has an inlet 74 and an outlet 76 which communicates to one another via the coolant passages 54. Connected to the electrode holder 78 via a sealing plate 83 by means of bolts 80 is an insulating end plate 82. The neutral electrode 42 and the end plate 82 have concentric bores 42b and 82a, respectively, in which a filter 84 is received to pass synthesis gas therethrough. The end plate 82 has a synthesis gas outlet 86.

The inlet 74 is connected to the feed water line 11 and the outlet 76 is connected to the feed water supply port 52. Feed water is preheated in the cooling section 63 and is discharged from the outlet 76 into the feed water supply port 52. Feed water is then introduced into the steam generating section 34A to form plasma gas composed of steam. A portion of the synthesis gas emitting from the outlet 86 may be recycled through a synthesis gas recirculation line (not shown) into the plasma reaction chamber 34 in which the water shift reaction takes place in the manner expressed by the reaction formula (2) described above. Designated at 88 is a seal member.

In FIG. 2, the electrode holder 28 fixedly supports three phase electrodes 36, 38, 40 which are supplied with alternating three phase electric power from the arc discharge power supply 16. The neutral electrode 42 is connected to a neutral point of the three phase arc power supply 16, which provides electric power output of output voltage in a value ranging from 30 to 240 Volts at an output frequency of 10 to 60 Hz.

In operation, the heat exchanger (not shown) is start up to maintain the methanation reactor MR at the temperature of 250 to 500°C. During this time period, the arc discharge electric power is supplied to the arc electrodes of the arc plasma reactor APR while the screw feeder 22 and the rotary valve 24 are driven to feed the solid carbon material to the arc plasma reactor APR. Next, the feed water supply pump P1 is driven to supply feed water to the steam generating zone 34A of the plasma reaction chamber 34 from the feed water supply port 52, with feed water being exposed to the high temperature to generate steam as plasma gas. Steam flows through the large number of minute plasma passages 35, with steam reacting with the solid carbon material at the temperature of more than 1000°C to be converted into synthesis gas with $H_2/Co$ ratio of more than 3. Synthesis gas is then cooled in the heat exchanger H and is further cooled in the cooler C1 to the temperature in the range between 60 to 90 °C. Synthesis gas thus cooled is supplied to the liquid/gas separator S 1 where moisture component is separated from synthesis gas as condensed water. When condensed water reaches a given level, the pump P2 is driven to supply condensed water to the feed water supply line 11 to be admixed with feed water. Mixed water is preheated at the cooling section 63 of the arc plasma reactor APR and is then supplied to the feed water supply port 52. On the other hand, synthesis gas is pressurized at the pressure level of about 15 to 50 atm by the compressor CM and is introduced into the methanation reactor MR, which is maintained at the temperature of 250 to 500°C, thereby converting synthesis gas into methane as expressed by the following formula:

$$CO + 3H_2 \rightarrow CH_4 + H_2O \qquad (3)$$

$$CO_2 + 4H_2 \rightarrow CH_4 + 2H_2O \qquad (4)$$

As previously noted, condensed water obtained in the above formulae (3) and (4) are recycled to the arc plasma reactor APR for producing the synthesis gas to lower the production cost of the heavier hydrocarbons and to eliminate environmental pollutions.

The methanation catalyst to be filled in the reactor MR may be of any type disclosed, for example, in US Patent Nos. 4,238,371, 4,368, 142, and 4,774, 261 and Japanese Patent Provisional Publication No. 5-184,925. Methane is cooled at the cooler C2 and is supplied through the expansion valve V to the liquid/gas separator S2 where condensed water is separated from the methane, with condensed water being recirculated from the bottom of the liquid/gas separator S2 to the feed water supply line 11 via a recycle line R and the circulation pump P2 to be recycled to the arc plasma reactor APR.

As previously noted, the methane is delivered through the flow control valves CV1, CV2 to form a mixture of methane and acetylene which is synthesized by the acetylene reactor AS. The mixture of methane and acetylene is introduced into the solid superacid reactor SC, by which isobutene is produced. The isobutene is then oligomerized in the oligomerization reactor PC to produce heavier hydrocarbons containing gasoline-range and jet fuel-range liquid fuels, offgas.

The system and method of the present invention provides numerous advantages over the prior art practices and which includes:

(1) Feed water and solid carbon materials, which are extremely low in cost, can be utilized as the raw materials, resulting in a remarkable reduction in production cost of heavier hydrocarbons. That is, the use of a technology of producing methane and acetylene from the solid carbon materials and feed water with the use of the synthesis gas generator, the methanation reactor and the acetylene reactor each of which is simple in construction and has an extremely high operating performance, enables methane and acetylene, that serves as raw material for the heavier hydrocarbons, to be produced in economically simplified steps to allow a combined use of the solid superacid catalyst reactor and the oligomerization reactor to produce extremely clean (without sulfur) heavier hydrocarbons at the maximum operating efficiency on a mass production basis.

(2) The utilization of arc plasma reactor which is small in structure but has a high operating performance enables efficient production of synthesis gas from the low cost solid carbon materials and feed water in a large volume to produce methane and acetylene at the highest production efficiency for thereby increasing the production efficiency of the clean heavier hydrocarbons that contain no sulfur.

(3) Since the carbon material is consumed only for producing synthesis gas and no carbon material is used as fuel for the reformer as would required in the prior art practice, the utilization rate of the carbon material is extremely high that leads to a remarkable reduction in production cost of the clean heavier hydrocarbons.

(4) Since the $H_2/CO$ ratio of synthesis gas in the arc plasma reactor can be easily adjusted to a suitable value optimum for the maximum operating performance in producing the synthesis gas by controlling the operating temperature of the arc plasma reactor APR, it is possible for the synthesis gas generator to be controlled in operation to provide an optimum operating control.

(5) Although the prior art practice needs a complex process for intermittently supply air into the reformer to obtain the synthesis gas while interrupting the synthesis gas production, the system and the method of the present invention do not require such a complex procedure for thereby simplifying the control procedure of producing the synthesis gas while enabling a remarkable reduction in a total production cost of the clean heavier hydrocarbons.

(6) In the prior art practice, since the reformer adopts the combustion method for producing synthesis gas, it is difficult for the reformer to control the operating temperature according to the operating condition of the synthesis gas generator at a high speed response. On the contrary, the present invention enables the arc plasma reactor to be precisely controlled at an appropriate temperature by merely varying an output voltage of the arc discharge power supply to be applied to the arc electrodes, providing a quick response to enable a mass production of the synthesis gas at the maximum efficiency for thereby enabling the heavier hydrocarbons to be produced at the maximum operating efficiency at an extremely low cost..

(7) In the prior art practice, condensed water obtained during production of the synthesis gas and during distilling stage of the hydrocarbons is expelled outside, causing environmental contaminants. On the contrary, the method and system of the present invention enables condensed water to be recycled as recycle water which is delivered to the arc plasma reactor APR, with a resultant remarkable decrease in the amount of feed water while eliminating environmental pollution.

(8) In the reforming process of natural gas to produce synthesis gas using partial combustion method, it takes a longer rise time and a longer dwell time. In contrast, the presence of capability of instantaneously producing synthesis gas by supplying electric power to the arc electrodes allows the synthesis gas generator to be started up and terminated in operation in quick response. This is especially advantageous for an improved operating efficiency and for an emergency stop such as earth quake.

(9) In the prior art practice, the synthesis gas generator of the hydrocarbon production system has a remarkably large size in a whole structure which increases running cost, thus requiring a sizable financial investment for such a plant. In contrast, the synthesis gas generator of the hydrocarbon production system of the present invention is small in size but high in operating efficiency and, therefore, there is no need for the sizable investment.

While a specific embodiment of the invention has been described in detail, it will be appreciated by those skilled in the art that various modifications and alternatives to those details could be developed in light of the overall teachings of the disclosure. Accordingly, the particular embodiment disclosed is meant to be illustrative only and not limiting to the scope of invention which is defined in appended claims.

**Claims**

1. A method of producing heavier hydrocarbons from solid carbon materials and feed water, comprising the steps of:

   preparing an arc plasma reactor having a plasma reactor chamber and arc discharge electrodes located in the reactor chamber;
   supplying the solid carbon materials into the reactor chamber to form a large number of minute arc passages in the solid carbon materials;
   supplying electric power to the arc electrodes to produce arc discharge plasmas in the minute arc passages, respectively;
   passing steam through the minute arc passages to react with the solid carbon materials under the presence of the arc discharge plasmas to produce synthesis gas containing $H_2$ and CO;
   introducing the synthesis gas into a methanation catalyst of a methanation reactor to synthesize methane;
   preparing first and second streams of the methane;
   converting the second stream of the methane into a stream of acetylene;
   preparing a mixture of the first stream of the methane and the stream of acetylene;
   reacting the mixture of the methane and the acetylene in the presence of a solid superacid catalyst to form isobutene; and
   converting the isobutene product in the presence of oligomerization catalyst into hydrocarbons.

2. The method of claim 1, wherein the thermal plasma reactor has an upstream side formed with a steam generating zone and a downstream side formed with a synthesis gas generating zone, and further comprising the steps of:

   supplying feed water into the steam generating zone of the arc plasma reactor to form the steam at the upstream side thereof;
   cooling the methane to separate condensed water; and
   recycling the condensed water into the steam generating zone to be converted into the steam.

3. The method of claims 1 or 2, further comprising the steps of:

   distilling the hydrocarbons to separate condensed water from the hydrocarbons;
   recycling the condensed water, obtained in the distilling step, to the steam generating zone of the arc plasma reactor to produce the steam; and

reacting the steam in the synthesis gas generating zone to produce the synthesis gas.

4. A heavier hydrocarbon production system comprising:

an arc plasma reactor having a solid carbon supply port, a feed water supply port, an insulating casing formed with a synthesis gas outlet, an arc plasma chamber formed in the insulating casing, arc discharge electrodes located in the arc plasma chamber, and a plurality of minute arc passages formed in solid carbon materials filled in the arc plasma chamber;

a feed water supply pump for supplying feed water into the arc plasma chamber via the feed water supply port to cause the feed water to be converted into steam;

an arc power supply for supplying electric power to the arc discharge electrodes to cause arc discharge plasmas to be generated in the minute arc passages, respectively, such that the water is exposed to the arc discharge plasmas to form the steam which reacts with the solid carbon materials in the presence of the arc discharge plasmas during passing through the minute arc passages to produce synthesis gas containing $H_2$ and CO; and

a methanation reactor having a methanation catalyst for converting the synthesis gas into methane;

an acetylene synthesizing reactor for converting a portion of a stream of the methane into acetylene;

a solid superacid catalyst reactor for reacting a mixture of the methane and the acetylene to synthesize isobutene; and

an oligomerization reactor for oligomerizing the isobutene into heavier hydrocarbons.

5. The heavier hydrocarbon production system of claim 4, further comprising:

a liquid/gas separator unit coupled to the methanation reactor for the methane and condensed water; and

a recycle line for recycling the condensed water to the arc plasma reactor to form the synthesis gas therein.

6. The heavier hydrocarbon production system of claims 4 or 5, further comprising:

a distillation tower for distilling the hydrocarbons to separate condensed water from the hydrocarbons;

a recycle line for recycling the condensed water, obtained in the distillation tower, to the arc plasma reactor to produce the synthesis gas.

# FIG. 1

# FIG. 2

**European Patent Office**

## EUROPEAN SEARCH REPORT

Application Number

EP 02 25 0601

## DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| A,D | US 5 723 505 A (CHAUMETTE PATRICK ET AL) 3 March 1998 (1998-03-03) * claims * | 1-6 | C07C1/04 C07C2/04 C07C2/76 C10L3/06 C10J3/00 H05H1/00 |
| A,D | US 4 973 776 A (ALLENGER VINCENZA M ET AL) 27 November 1990 (1990-11-27) * claims * | 1-6 | |
| A | WO 89 11448 A (LEWIS ARLIN C) 30 November 1989 (1989-11-30) * claims; figure * | 1-6 | |
| A | US 4 606 799 A (PIRKLBAUER WILFRIED ET AL) 19 August 1986 (1986-08-19) * claims * | 1-6 | |

TECHNICAL FIELDS
SEARCHED     (Int.Cl.7)

C07C
C10L
C10J

The present search report has been drawn up for all claims

| Place of search | Date of completion of the search | Examiner |
|---|---|---|
| MUNICH | 16 May 2002 | Seufert, G |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**EP 1 227 074 A1**

## ANNEX TO THE EUROPEAN SEARCH REPORT
## ON EUROPEAN PATENT APPLICATION NO.

EP 02 25 0601

This annex lists the patent family members relating to the patent documents cited in the above-mentioned European search report.
The members are as contained in the European Patent Office EDP file on
The European Patent Office is in no way liable for these particulars which are merely given for the purpose of information.

16-05-2002

| Patent document cited in search report | | Publication date | Patent family member(s) | | Publication date |
|---|---|---|---|---|---|
| US 5723505 | A | 03-03-1998 | FR | 2719054 A1 | 27-10-1995 |
| | | | AU | 698967 B2 | 12-11-1998 |
| | | | AU | 1763995 A | 02-11-1995 |
| | | | CA | 2147809 A1 | 26-10-1995 |
| | | | DE | 69522988 D1 | 08-11-2001 |
| | | | DE | 69522988 T2 | 04-04-2002 |
| | | | EP | 0679709 A1 | 02-11-1995 |
| | | | NO | 951525 A | 26-10-1995 |
| | | | ZA | 9503283 A | 24-10-1996 |
| US 4973776 | A | 27-11-1990 | CA | 1304419 A1 | 30-06-1992 |
| WO 8911448 | A | 30-11-1989 | AT | 121374 T | 15-05-1995 |
| | | | AU | 617486 B2 | 28-11-1991 |
| | | | AU | 3686889 A | 12-12-1989 |
| | | | CA | 1339271 A1 | 12-08-1997 |
| | | | DE | 68922310 D1 | 24-05-1995 |
| | | | DE | 68922310 T2 | 12-10-1995 |
| | | | DK | 278990 A | 23-11-1990 |
| | | | EP | 0425506 A1 | 08-05-1991 |
| | | | IN | 167224 A1 | 22-09-1990 |
| | | | JP | 2811593 B2 | 15-10-1998 |
| | | | JP | 3505570 T | 05-12-1991 |
| | | | MX | 170703 B | 08-09-1993 |
| | | | WO | 8911448 A1 | 30-11-1989 |
| | | | US | 5069765 A | 03-12-1991 |
| US 4606799 | A | 19-08-1986 | AT | 384007 B | 25-09-1987 |
| | | | AT | 111084 A | 15-02-1987 |
| | | | AU | 573314 B2 | 02-06-1988 |
| | | | AU | 3981185 A | 10-10-1985 |
| | | | CA | 1232229 A1 | 02-02-1988 |
| | | | DD | 232065 A5 | 15-01-1986 |
| | | | DE | 3572993 D1 | 19-10-1989 |
| | | | EP | 0157758 A2 | 09-10-1985 |
| | | | JP | 1953893 C | 28-07-1995 |
| | | | JP | 6076586 B | 28-09-1994 |
| | | | JP | 60226596 A | 11-11-1985 |
| | | | ZA | 8502084 A | 27-11-1985 |

EPO FORM P0459

For more details about this annex : see Official Journal of the European Patent Office, No. 12/82

11